(19)

Europäisches
Patentamt
European
Patent Office
Office européen
des brevets

(11) **EP 2 314 262 A1**

(12) **EUROPEAN PATENT APPLICATION**

(43) Date of publication:
**27.04.2011 Bulletin 2011/17**

(51) Int Cl.:
**A61F 13/15** (2006.01)

(21) Application number: **10251786.9**

(22) Date of filing: **12.10.2010**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **13.10.2009 US 578003
13.10.2009 US 578028**

(71) Applicant: **Johnson & Johnson Industria e
Comercio Ltda.
12237-350 Sao Paulo (BR)**

(72) Inventors:
• **Wöhlke, Jonathan
Manaus, AM 69060-081 (BR)**
• **Bruzadin, Fernanda
São Paulo (BR)**
• **Franca Rangel, Fabio Eduardo
São Paulo (BR)**

(74) Representative: **Carpmaels & Ransford
One Southampton Row
London
WC1B 5HA (GB)**

(54) **Articles including functional benefit and a plurality of visually distinct fibers indicative of the functional benefit**

(57)    A nonwoven material and an absorbent article that provide a signal viewable from the top surface of the nonwoven material and absorbent article which is indicative of a functional benefit of the nonwoven and article. The signal is delivered by use of a plurality of visually distinct fibers that are visually distinct from an adjacent background color of the top surface.

**EP 2 314 262 A1**

**Description**

**FIELD OF INVENTION**

The present invention generally relates to an absorbent article including a functional benefit and a plurality of visually distinct colored fibers indicative of the functional benefit. The plurality of visually distinct colored fibers function to visually communicate to a user the functional benefit. The present invention also relates to nonwoven materials including a plurality of visually distinct fibers.

**BACKGROUND OF THE INVENTION**

Due to improvements in materials that constitute disposable absorbent articles, and improvements in the construction of such articles, commercially available sanitary disposable absorbent articles can provide excellent fluid handling properties. Further, it is known in the prior art to provide disposable absorbent articles with other functional properties. For example, it is known to provide the cover layer of such articles with a skin soothing agent to thereby provide a skin-soothing benefit to such articles. However, much of the technology that provides enhanced fluid handling properties, or other functional properties to the article (e.g. skin soothing), is hidden from the consumer. Consequently, these enhanced functional properties are not readily communicated to the user, visually or otherwise.

The inventors of the present invention have discovered a way to visually communicate functional characteristics of a disposable absorbent article to a user by incorporating visually distinct colored fibers into the article that are viewable from a top surface of the article. The fibers function to visually communicate the enhanced functional properties of the article to a user. The inventors also disclose herein nonwoven materials including a plurality of visually distinct fibers for visually communicating functional properties of the nonwoven material to a user.

**SUMMARY OF THE INVENTION**

In view of the above the present invention provides a nonwoven material and an absorbent article including a top surface wherein the top surface includes at least one background color and a plurality of visually distinct fibers, the visually distinct fibers functioning to visually convey a functional benefit of the nonwoven and article to a user. The absorbent article may comprise the nonwoven material.

**BRIEF DESCRIPTION OF THE DRAWINGS**

Examples of embodiments of the present invention will now be described with reference to the drawings, in which:

Fig. 1 is a top perspective view of an absorbent article according to the present invention;
Fig. 2 is a bottom perspective view of the absorbent article shown in Fig. 1;
Fig. 3 is an exploded view of the absorbent article shown in Fig. 1;
Fig. 4 is a detailed perspective of a portion of the absorbent article depicting a plurality of visually distinct fibers viewable from a top surface of the article; and
Fig. 5 is a detailed view of a portion of a non-woven material forming the cover layer of the article shown in Fig. 1, such detailed portion identified by the circle shown in Fig. 3.

**DETAILED DESCRIPTION OF THE INVENTION**

The present invention generally relates to disposable absorbent articles such as tampons, sanitary napkins, pantiliners, absorbent products for incontinence, baby diapers, and other disposable absorbent articles adapted to absorb body exudates. "Disposable absorbent articles" for purposes of the present invention shall also include wipes. Although the invention will be described herein with reference to a sanitary napkin, the invention may be utilized with other disposable absorbent articles.

The term color as used herein includes any color in the visual spectrum, including white, black, red, blue, violet, orange, yellow, green, indigo, as well as any mixture or combination thereof.

The term "disposable" as used herein to describe absorbent articles that are not intended to be laundered or otherwise reused as an absorbent article.

The term "visually distinct fiber(s)" as used herein means a fiber that: (1) is of a sufficient size to be individually visible to a user having 20/20 vision from a top surface of a disposable absorbent article, or nonwoven, at a distance of twelve inches; and (2) is visually distinguishable from a "directly adjacent background color" (as defined herein) of the top surface of the article, or nonwoven, to a user having 20/20 vision at a distance of twelve inches; and (3) does not intersect with any other fiber meeting the criteria of (1) and (2).

The visually distinct fibers employed in the sanitary absorbent articles of the present invention are used to convey to the user a functional benefit of the article that would otherwise be invisible to the user. For example, sanitary absorbent articles may be provided with a skin soothing ingredient. Normally, such a skin soothing agent would be invisible to the user and as such the user may not be aware of the presence of such skin soothing agent prior to use of article. However, the presence of the visually distinct fibers in the absorbent articles of the present invention function to provide a visual cue to the user as to the presence of the skin soothing agent. The functional benefit to be conveyed to the user may include an area of higher absorbent capacity, moisture control, skin soothing, fragrance, odor control, heating, cooling, anti-bacterial activity, anti-fungal activity, anti-microbial activity, protease and/or enzyme inhibiting activity, redness reduction, itch prevention, inflammation reduction, skin conditioning, pH control, softening, masking, freshness, vitamins, cleansing, depilatory, anti-acne, anti-wrinkle, topical anesthetic, artificial tanning, anti-viral, sunscreen properties, anti-oxidants, skin exfoliation and the like, and a combination of one or more of these characteristics.

The present invention also relates to nonwoven materials including a plurality of visually distinct fibers that are visible from a top surface of the nonwoven material, the plurality of visually distinct fibers functioning to visually convey at least one of the functional benefits set forth above. Nonwoven materials according to the present invention are particularly suited for use in disposable absorbent articles.

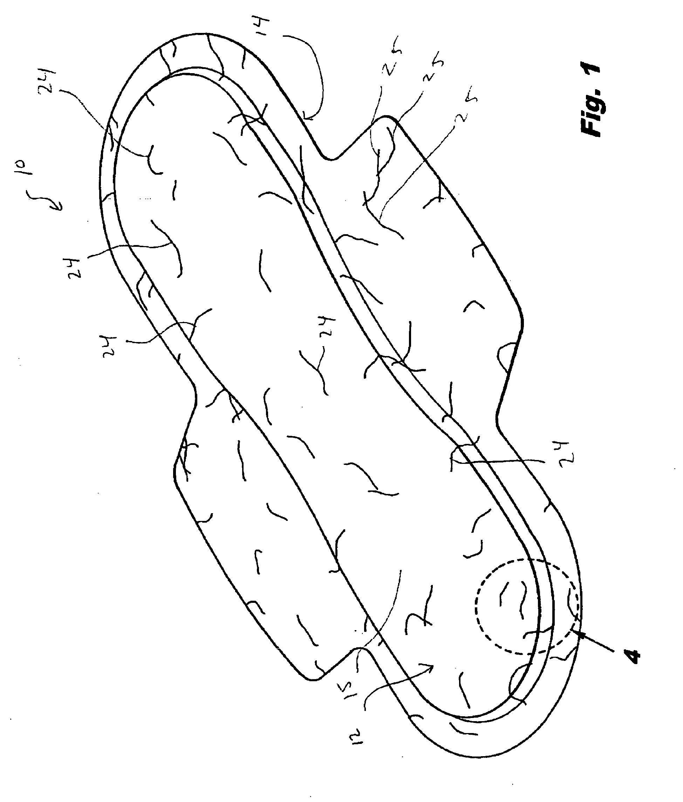

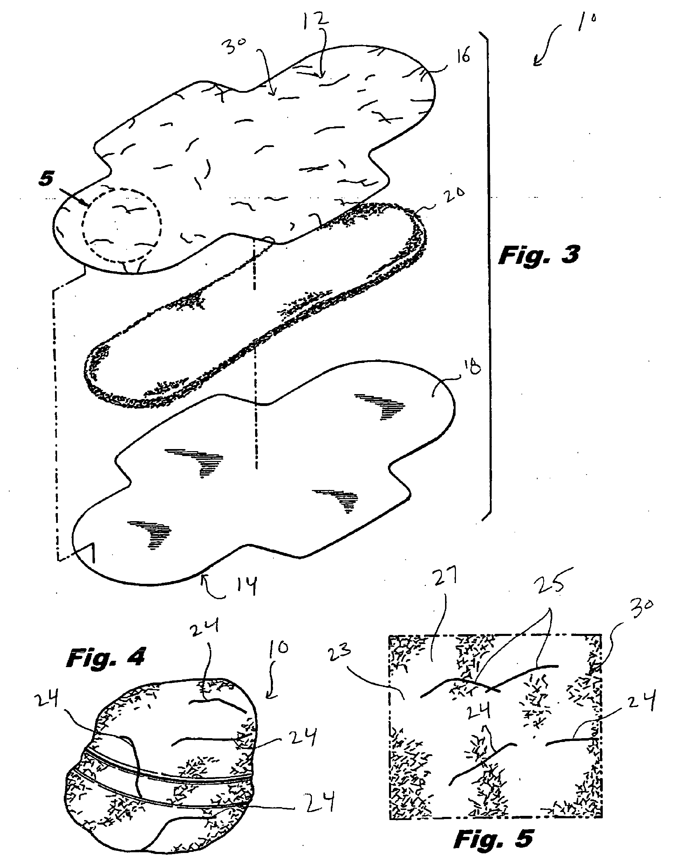

Fig. 1 provides a perspective view of a sanitary napkin 10 in accordance with one embodiment of the present invention. The sanitary napkin 10 includes a top surface 12 and a bottom surface 14. As shown in Fig. 3, the sanitary napkin 10 includes a liquid permeable cover layer 16, a liquid impermeable barrier layer 18, and an absorbent core 20 arranged between the cover layer 16 and the barrier layer 18. The sanitary napkin 10 may optionally include a transfer layer (not shown) arranged between the cover layer 16 and the core 20.

In the embodiment of the invention show in Fig. 1 the top surface of the sanitary napkin 12 includes a background color 15 viewable from the top surface 12 of the napkin 10 and a plurality of visually distinct fibers 24 that are viewable from the top surface 12 of the napkin 10.

The visually distinct fibers 24 may be any color or combination of colors. In addition, individual fibers 24 may be a different color than other fibers 24. For example, some fibers 24 may be blue and other fibers 24 may be green. However, in order for the visually distinct fibers 24 to be visible to user having 20/20 vision at a distance of twelve inches from the top surface 12 of the napkin each of the fibers 24 must be colored a sufficiently different color than a "directly adjacent background color" of the top surface 12 of the napkin 10. The "directly adjacent background color" is the color of that portion of the top surface 12 of the napkin 10 that is directly adjacent to the individual fiber 24 being viewed. Thus, in the embodiment of the invention shown in Fig. 1, the "directly adjacent background color" would be that portion of the background color 15 that is directly adjacent an individual fiber 24. In preferred embodiments of the present invention the fibers 24 have a difference in color of at least $\Delta E^* > 3$, more preferably $\Delta E^* > 5$, relative to the directly adjacent background color, as measured according to the test method set forth below.

The background color may be any color, or combination of colors, provided that the directly adjacent background color is sufficiently different from the color of the fibers 24 to enable the fibers 24 to be visible from the top surface to a user having 20/20 vision at a distance of twelve inches the napkin. The background color may be provided exclusively by the color of the material(s) defining the top surface 12 of the napkin 10. Alternatively, the background color of the top surface may be provided by a separate process for imparting color to the top surface 12 of the napkin 10, such as printing or the like. In other embodiments the background color may be delivered by providing a colored layer or insert below the cover layer 16 of the napkin 10 and selecting a cover material that enables the background color to be visible through the cover layer 16 from the top surface 12 of the napkin 10.

In a preferred embodiment of the invention the visually distinct fibers 24 are incorporated into a nonwoven material 30 as show in FIG. 5. As show in FIG. 5 the visually distinct fibers 24 are visible from a top surface 27 of the nonwoven material 30. The fibers 24 are colored such that they have a difference in color from a directly adjacent background color 23 of the nonwoven 30 in the same manner as discussed above with respect to the sanitary napkin 10.

As shown in FIG. 5, the visually distinct fibers 24 are incorporated into the fiber mixture of the nonwoven material 30. In preferred embodiments the visually distinct fibers 24 comprise between 1% and 15% by weight, and more preferably between 2% and 5% by weight, of fiber mixture of the nonwoven material 30. Preferably, each of the visually distinct fibers 24 has a length of between 5 mm and 40 mm and a linear density of between 0.5 dtex and 50 dtex, and more preferably from 15 to 35 dtex.

Other than the visually distinct fibers 24, the fiber mixture of the nonwoven material 30 may be composed of only one type of fiber, such as polypropylene, or it may include a mixture of more than one fiber. The nonwoven material 30 may be composed of bi-component or conjugate fibers having a low melting point component and a high melting point component. The fibers forming the nonwoven may be selected from a variety of natural and synthetic materials such as polypropylene, polyethylene, nylon, polyester, rayon (in combination with other fibers), cotton, acrylic fiber and the like and combinations thereof. The fibers forming the nonwoven, other than the visually distinct fibers, preferably have length in the range of between 30 mm and 50 mm.

In one embodiment of the invention, a ratio of the linear density of the visually distinct fibers relative to the linear density of the other nonwoven fibers forming the nonwoven is preferably in the range of 1 to 100, and more preferably in the range of 5-40.

The nonwoven material 30 may be manufactured using any suitable nonwoven manufacturing technique known to those of skill in the art. For example, the nonwoven may be made by means of extrusion of fibers that are first cut to desired lengths from long strands, carded and bonded together by a thermal calendar in a thermobonding process. Alternatively, the nonwoven may be formed by stitchbonding, needlepunching, hydroentangling, latex bonding, hot through air bonding, spunlace, meltblown, air laid, wet laid or other suitable nonwoven process.

Referring to Fig. 1 and Fig. 5, it is noted that some of the colored fibers overlap or intersect with other colored fibers. For example, the fibers identified by the reference numeral 25 in Fig. 1 and Fig. 5 intersect one another. Since these fibers 25 intersect one another, then these fibers are not "visually distinct fibers" as defined herein. However, it is not required that every colored fiber of a given article or nonwoven be a "visually distinct fiber" in order for such article or nonwoven to be within the scope of the present invention. Rather, it is merely required that such article or nonwoven includes at least a plurality of such "visually distinct fibers" as defined herein.

In a preferred embodiment of the invention, the nonwoven material 30 is used as the cover layer 16 of the napkin 10. If the nonwoven material 30 is used as the cover layer 16, the nonwoven material 30 may be treated with a surfactant to impart the desired degree of wettability to the cover layer 16. Alternatively, the nonwoven material 30 may be used as an insert arranged below a separate cover layer 16, provided that that cover layer 16 is a material of sufficient transparency to thereby enable the fibers 24 to be viewed through the cover layer 16.

In one embodiment, the absorbent core 20 is a blend or mixture of cellulosic fibers and superabsorbent disposed therein. Cellulosic fibers that can be used in the absorbent core 20 are well known in the art and include wood pulp, cotton, flax and peat moss. Wood pulp is preferred. Pulps can be obtained from mechanical or chemimechanical, sulfite, kraft, pulping reject materials, organic solvent pulps, etc. Both softwood and hardwood species are useful. Softwood pulps are preferred. It is not necessary to treat cellulosic fibers with chemical debonding agents, cross-linking agents and the like for use in the present material. Some portion of the pulp may be chemically treated as discussed in U.S. Pat. No. 5,916,670 to improved flexibility of the product. Flexibility of the material may also be improved by mechanically working the material or tenderizing the material.

The absorbent core 20 can contain any superabsorbent polymer (SAP) which are well known in the art. For the purposes of the present invention, the term "superabsorbent polymer" (or "SAP") refers to materials which are capable of absorbing and retaining at least about 10 times their weight in body fluids under a 0.5 psi pressure. The superabsorbent polymer particles of the invention may be inorganic or organic crosslinked hydrophilic polymers, such as polyvinyl alcohols, polyethylene oxides, crosslinked starches, guar gum, xanthan gum, and the like. The particles may be in the form of a powder, grains, granules, or fibers. Preferred superabsorbent polymer particles for use in the present invention are crosslinked polyacrylates, such as the product offered by Sumitomo Seika Chemicals Co., Ltd. Of Osaka, Japan, under the designation of SA70N and products offered by Stockhausen Inc.. In a specific example, the absorbent core is a material containing from 95% to about 40% percent cellulosic fiber by weight, and about 5% to about 60% SAP by weight.

If a transfer layer is employed in the napkin 10, then such transfer layer is arranged adjacent to an inside surface of the cover layer 16. The transfer layer provides the means of receiving body fluid from the cover layer 16 and holding it until the absorbent core 20 has an opportunity to absorb the fluid, and therefore serves as a fluid transfer or acquisition layer. In addition the transfer layer functions to wick the fluid in the longitudinal and transverse directions of the napkin so that the full absorbent capacity of the napkin is utilized.

The transfer layer is, preferably, more dense and has a larger proportion of smaller pores than the cover layer 16. These attributes allow the transfer layer to contain body fluid and hold it away from the outer side of the cover layer 16, thereby preventing the fluid from re-wetting the cover layer 16 and its surface.

The transfer layer may be composed of fibrous materials, such as wood pulp, polyester, rayon, flexible foam, or the like, or combinations thereof. Preferably, the transfer layer is free of any superabsorbent polymer (SAP). The transfer layer may also comprise thermoplastic fibers for the purpose of stabilizing the layer and maintaining its structural integrity. The transfer layer may be treated with surfactant on one or both sides in order to increase its wettability, although generally the transfer layer is relatively hydrophilic and may not require treatment. The transfer layer is preferably bonded on both sides to the adjacent layers, i.e. the cover layer 16 and the barrier layer 18.

Underlying the absorbent core 20 is a barrier layer 18 comprising liquid-impervious film material so as to prevent liquid from egressing the sanitary napkin and staining the wearer's undergarment. The barrier layer 18 is preferably made of polymeric film, although it may be made of liquid impervious, air-permeable material such as repellent-treated non-woven or micropore films or foams.

The barrier layer 18 may be breathable, i.e., permits vapor to transpire. Known materials for this purpose include nonwoven materials and microporous films in which microporosity is created by, inter alia, stretching an oriented film. Single or multiple layers of permeable films, fabrics, melt-blown materials, and combinations thereof that provide a tortuous path, and/or whose surface characteristics provide a liquid surface repellent to the penetration of liquids may also be used to provide a breathable backsheet. The cover layer 16 and the barrier layer 18 are preferably joined along their marginal portions so as to form an enclosure or flange seal that maintains the absorbent core 20 captive. The joint may be made by means of adhesives, heat-bonding, ultrasonic bonding, radio frequency sealing, mechanical crimping, and the like and combinations thereof.

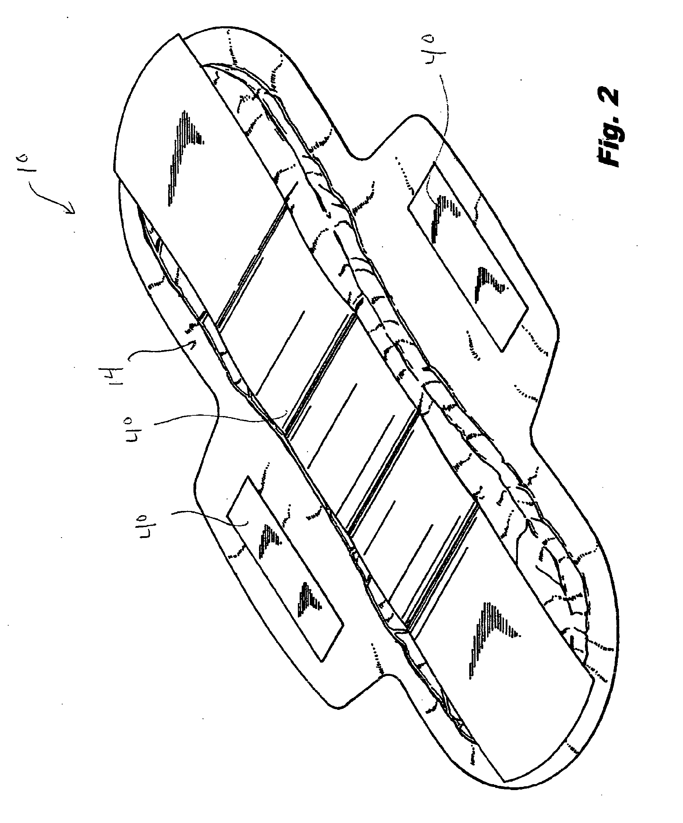

Positioning adhesive may be applied to the bottom surface 14 of the napkin 10 for securing the napkin 10 to a garment during use. As seen in Fig. 2, the positioning adhesive may be covered with removable release paper 40 so that the positioning adhesive is protected by the removable release paper 40 prior to use.

If the nonwoven material 30 according to the present invention is employed in a wipe, the nonwoven material 30 preferably forms the body-facing layer of such wipe. If the nonwoven material 30 is employed in a tampon, the nonwoven material 30 is preferably employed to define the outer surface of the tampon.

**Procedure for Measuring Color Difference of Visually Distinct Fibers and Directly Adjacent Background Color**

The L*, a* and b* values for the inventive articles described herein are measured using the widely accepted CIE LAB scale. In the CIE LAB system, each color is defined based upon three values L*, a* and b*. The L* value is a measure of light reflectance wherein absolute black is at the bottom of the scale (L*=0) and absolute white is at the top of the scale (L*=100), a* represents the presence of green or red in the specific color (+a* = red, -a*=green), and b* represents the presence of yellow or blue in the specific color (+b*=yellow, -b*=blue). The L*, a* and b* each define an axis within the CIE LAB system. Therefore, each specific color is defined as a point within the three dimensional space defined by the L* axis, a* axis, and b* axis.

According to the CIE LAB system the difference in color between two specific colors can be mathematically expressed according to the following equation:

$$\Delta E^* = [(L^*{}_x - L^*{}_y)^2 + (a^*{}_x - a^*{}_y)^2 + (b^*{}_x - b^*{}_y)^2]^{1/2}$$

In the above equation, $L^*{}_x$, $a^*{}_x$ and $b^*{}_x$ represent the first color and $L^*{}_y$, $a^*{}_y$ and $b^*{}_y$ represent the second color, thus $\Delta E^*$ represents the relative difference in color between the two colors.

As previously noted, in preferred embodiments of the present invention, the fibers 24 have a difference in color of $\Delta E^* > 3$, more preferably $\Delta E^* > 5$, relative to the directly adjacent background color, as measured according to the test method set forth below.

To measure the L*, a* and b* values for absorbent articles and nonwoven materials according to the present invention the following procedure is employed.

Equipment - Digital SLR Camera, for example a Canon Rebel xTi or equivalent, digital camera software with appropriate computer system, Broncolor Picolite flash system with two flashes or equivalent, Gregtag Macbeth colorchecker chart, Software - Corel Paint Shop Pro Photo XI or equivalent, and a copy stand.

Equipment Set up and Measurement - Set up the copy stand to assure that the absorbent article is in view and no more than half of the absorbent article will be in the photograph. If an absorbent article is being analyzed the absorbent article should be unwrapped, unfolded an arranged such that the body facing surface of the article is facing the camera. If a nonwoven material, i.e. independent of an article, is being analyzed the surface of the nonwoven that more clearly shows the visually distinct fibers should be arranged facing the camera. In addition, for a nonwoven material, two pieces of plain white copy paper, such as OfficeMax® 20 1b.weight copy paper, 92/104 US/Euro brightness, commercially available from OfficeMax North America, Inc., Naperville, Illinois, should be arranged under the nonwoven material prior to taking any measurements.

Use the "auto-focus" feature of camera to set the focus. Assure that the flash system is activated by the camera system. The system should be set on 5.9 for both flashes. Standard room lighting is acceptable. Be sure that no unusual lighting is present.

Assure camera focus is locked on that setting and in a manual mode. Suggested camera settings are ISO = 100, Speed = 1/60 and Aperture = 16.

Take a photo of the Gregtag Macbeth Colorchecker Chart, where the neutral white chip is in the center of the photo. Using Paint Shop Pro, open the Photo of the Colorchecker Chart and use the "eyedropper" tool to point to the appropriate white chip (usually the "whitest chip") and record the "R", "G" and "B" Values. The target is for all three to be at 240 ± 2. If the target is met, the apparatus is appropriately calibrated. If the target is not met, adjust the camera aperture to achieve this target. Adjustment of the ISO can also be done if necessary, however adjustment of the camera aperture should be attempted first. Save the photo file and record the "R", "G" and "B" values.

Position the absorbent article or nonwoven and assure that you are using most of the available camera viewing area. Take a photograph and record the file name or rename the file for convenience.

Using Corel Paint Shop Pro Photo XI or equivalent software, open the photo in Photoshop - be sure you can see the full photo, then magnify the photo to clearly see a visually distinct fiber, be sure the fiber is multiple pixels "thick".

Survey the photo to locate the darkest fiber that does not overlap or intersect another fiber. Using the "eyedropper" tool, point to a fiber in the photo. Try to be in the middle of the fiber. Record the "L*", "a*" and "b*" values for the most distinctly visible fiber in the photo. Repeat the previous steps to collect at least four sets of data for the selected fiber.

Using the "eyedropper" tool, point to the area directly adjacent the fiber measured above and record the "L*", "a*" and "b*" values for this "directly adjacent background color". Repeat the previous steps to collect at least four sets of data for the directly adjacent background color. Average "L*", "a*" and "b*" values for the fiber. Average the "L*", "a*" and "b*" values for the directly adjacent background color. Calculate the $\Delta E^*$ for the averaged L*, a*, b* readings according the following equation:

$$\Delta E^* = [(L^*_1 - L^*_2)^2 + (a^*_1 - a^*_2)^2 + (b^*_1 - b^*_2)^2]^{1/2}$$

where (L*), $a^*_1$, and $b^*_1$)= Visually Distinct Fiber Color; and
where (L*$_2$, a*$_2$, b*$_2$) = Directly Adjacent Background Color.

## EXAMPLES

### Inventive Example # 1

An inventive example of a nonwoven material according to the present invention was constructed as follows. A first plurality of polypropylene fibers were extruded from a polypropylene feed to provided fibers having a linear density of 0.9 dtex. The fibers were cut such that fifty percent of the fibers, by weight, had a length of 30 mm and fifty percent of the fibers, by weight, had a length of 40 mm. The first plurality of fibers were provided with a white color by adding 2% by weight of $TiO_2$ to the polymer feed. The white fibers functioned to provide the background color of the nonwoven. The first plurality of fibers were provided with a skin soothing benefit by adding 0.5 % of a fiber finish including aloe vera to the polymer feed.

A second plurality of polypropylene fibers were extruded from a polypropylene feed, to provide fibers having a linear density of 18dtex. The second plurality of fibers were cut to have a length of 30 mm. The second plurality of fibers were provided with a blue color by adding 2% by weight of a master batch, commercially available from Cromex S/A, Sao Paulo, Brazil, under product code PE-AZ-24122, to the polymer feed. The blue fibers functioned as the visually distinct fibers of the nonwoven.

The first plurality of fibers (white) and second plurality of fibers (blue) were mixed such that the second plurality of fibers formed 3%, by weight, of the fibrous mixture. The fibrous mixture was fed into a conventional thermobonding nonwoven machine to form the final nonwoven material. The final nonwoven material had a basis weight of 16 gsm (g/m$^2$).

### Inventive Example #2

An inventive example of a sanitary napkin according to the present invention was constructed as follows. The sanitary napkin was constructed to a have a structure as depicted in Fig. 3. The cover layer of the napkin was formed from the nonwoven material described in Inventive Example #1 above. The absorbent core was constructed from 5.0 g of wood pulp commercially available from Georgia Pacific, Atlanta, Georgia, under product code 111410 and 0.4 g super absorbent polymer commercially available from Sumitomo Seika Ltd., Osaka, Japan, under product code SA70. The barrier layer was constructed from a polyethylene film material commercially available from Clopay Plastic Products, Mason, Ohio, under product code 113689. The cover layer was attached to the barrier by H-2900 adhesive from Bostik-Findley, Wauwatosa, WI.

### Inventive Example #3

An inventive example of the sanitary napkin according to the present invention was constructed in a substantially identical manner to the sanitary napkin described in Example 2 above. However, during formation of the nonwoven cover layer 20% by weight of Coolmax® fibers, commercially available from Invista, S.a.r.l., Wichita, Kansas, were added to the fibrous mixture prior to feeding such fibrous mixture to the thermobonding machine. The Coolmax fibers functioned to provide the article with a cooling effect.

**Inventive Example #4**

An inventive example of a nonwoven material according to the present invention was constructed in an essentially identical manner to the nonwoven material described in Example 1above. However, the second plurality of fibers were formed to have a linear density of 30 dtex. In addition, the first plurality of fibers (white) and second plurality of fibers (blue) were mixed such that the second plurality of fibers formed 2%, by weight, of the fibrous mixture.

The L*, a*, b* values of a visually distinct fiber and a directly adjacent background color is set forth in Table 1 for the Inventive Examples described above. The $\Delta E^*$ value indicating the difference in color between the visually distinct fibers and the directly adjacent background color is also set forth in Table 1.

**TABLE 1**

|  | L*, a*, b* Values of Visually Distinct Fiber | L*, a*, b* Values of Directly Adjacent Background Color | $\Delta E^*$ |
| --- | --- | --- | --- |
| Inventive Example #1 | 86, -3, -4 | 93, 0, -1 | 10 |
| Inventive Example #2 | 88, -5, -6 | 94, 0, 0 | 9 |
| Inventive Example #3 | 88, -5. -6 | 94, 0, 0 | 9 |
| Inventive Example #4 | 86, -3, -4 | 93, 0, -1 | 10 |

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of the invention.

**Claims**

1. A nonwoven material or an absorbent article comprising:

   a top surface;
   wherein the top surface includes at least one background color and a plurality of visually distinct fibers, the visually distinct fibers functioning to visually convey a functional benefit of the nonwoven to a user.

2. The nonwoven material according to claim 1, comprising:

   a top surface;
   wherein the top surface includes at least one background color and a plurality of visually distinct fibers, the visually distinct fibers functioning to visually convey a functional benefit of the nonwoven to a user.

3. The absorbent article according to claim 1, comprising:

   a top surface;
   wherein the top surface includes at least one background color and a plurality of visually distinct fibers, the visually distinct fibers functioning to visually convey a functional benefit of the article to a user.

4. The nonwoven material or absorbent article according to any one of the preceding claims, wherein the visually distinct fibers each have a color, and a portion of the background color directly adjacent each of the visually distinct fibers has a color, and wherein a difference in color between each visually distinct fiber and its corresponding directly adjacent background color is calculated according to the formula $\Delta E^* = [(L^*_1 - L^*_2)^2 + (a^*_1 - a^*_2)^2 + (b^*_1 - b^*_2)^2]^{1/2}$.

5. The nonwoven material or absorbent article according to claim 4, wherein difference in color between each visually distinct fiber and its corresponding directly adjacent background color is $\Delta E^* > 3$.

6. The nonwoven material or absorbent article according to claim 5, wherein difference in color between each visually distinct fiber and its corresponding directly adjacent background color is AE* > 5.

7. The nonwoven material or absorbent article according to any one of the preceding claims, wherein each visually distinct fiber has a length in the range of between 5 mm and 40 mm.

8. The nonwoven material or absorbent article according to any one of the preceding claims, wherein each visually distinct fiber has a linear density between 0.5 dtex and 50 dtex.

9. The nonwoven material or absorbent article according to claim 8, wherein each visually distinct fiber has a linear density between 15 dtex and 35 dtex.

10. The absorbent article according to any one of claims 1 and 3 to 9, wherein the absorbent article comprises a nonwoven material including the plurality of visually distinct fibers.

11. The nonwoven material or absorbent article according to any one of the preceding claims, wherein the nonwoven material includes between 1% and 15% by weight of the visually distinct fibers.

12. The nonwoven material or absorbent article according to claim 11, wherein the nonwoven material includes between 2% and 5% by weight of the visually distinct fibers.

13. The absorbent article according to any one of claims 1 and 3 to 12, wherein the visually distinct fibers visually convey a benefit of the article, the benefit selected from the group consisting of an area of higher absorbent capacity, moisture control, skin soothing, fragrance, odor control, heating, cooling, anti-bacterial activity, anti-fungal activity, anti-microbial activity, protease and/or enzyme inhibiting activity, redness reduction, itch prevention, inflammation reduction, skin conditioning, pH control, softening, masking, freshness, vitamins, cleansing, depilatory, anti-acne, anti-wrinkle, topical anesthetic, artificial tanning, anti-viral, sunscreen properties, anti-oxidants, skin exfoliation and the like, and a combination of one or more of these characteristics.

14. The absorbent article according to any one of claims 1 and 3 to 13, wherein the absorbent article is one of a tampon, sanitary napkin, pantiliner, incontinence article, baby diaper, and wipe.

15. The absorbent article according to any one of claims 1 and 3 to 14, wherein the absorbent article comprises:

   a liquid permeable cover layer;
   a liquid impermeable barrier layer;
   an absorbent core arranged between the barrier layer and the cover layer; and
   wherein the cover layer is formed from the nonwoven material.

*Fig. 1*

Fig. 2

**Fig. 3**

**Fig. 4**

**Fig. 5**

Europäisches
Patentamt

European
Patent Office

Office européen
des brevets

# EUROPEAN SEARCH REPORT

Application Number

EP 10 25 1786

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2005/066406 A1 (KB AVIAT INC [US]) 21 July 2005 (2005-07-21) * page 7, line 10 - line 19 * * page 7, line 28 - page 8, line 15 * * page 10, line 22 - line 26 * * figure 22 * * page 34, line 30 - line 33 * ----- | 1-10, 13-15 | INV. A61F13/15 |
| X | CN 1 335 424 A (CAO YIDONG [CN]) 13 February 2002 (2002-02-13) * abstract; figures 1-2; examples 1-2 * ----- | 1,2,4-9, 11,12 | |
| X | KR 2009 0020997 A (NEWMAN CO LTD [KR]) 27 February 2009 (2009-02-27) * abstract * ----- | 1-9, 13-15 | |

TECHNICAL FIELDS
SEARCHED      (IPC)

A61F

The present search report has been drawn up for all claims

| Place of search | Date of completion of the search | Examiner |
|---|---|---|
| Munich | 25 January 2011 | Mangin, Sophie |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**ANNEX TO THE EUROPEAN SEARCH REPORT
ON EUROPEAN PATENT APPLICATION NO.**

EP 10 25 1786

This annex lists the patent family members relating to the patent documents cited in the above-mentioned European search report.
The members are as contained in the European Patent Office EDP file on
The European Patent Office is in no way liable for these particulars which are merely given for the purpose of information.

25-01-2011

| Patent document cited in search report | | Publication date | Patent family member(s) | | Publication date |
|---|---|---|---|---|---|
| WO 2005066406 | A1 | 21-07-2005 | AT | 423863 T | 15-03-2009 |
| | | | AU | 2004312544 A1 | 21-07-2005 |
| | | | CA | 2551104 A1 | 21-07-2005 |
| | | | EP | 1709227 A1 | 11-10-2006 |
| | | | NO | 328084 B1 | 30-11-2009 |
| | | | NZ | 548591 A | 25-09-2009 |
| | | | US | 2005170726 A1 | 04-08-2005 |
| CN 1335424 | A | 13-02-2002 | NONE | | |
| KR 20090020997 | A | 27-02-2009 | NONE | | |

EPO FORM P0459

For more details about this annex : see Official Journal of the European Patent Office, No. 12/82

**EP 2 314 262 A1**

**REFERENCES CITED IN THE DESCRIPTION**

**Patent documents cited in the description**

- US 5916670 A